Europäisches Patentamt

European Patent Office

Office européen des brevets

(11) Publication number: **0 178 352**
**B1**

# EUROPEAN PATENT SPECIFICATION

(45) Date of publication of patent specification: **20.12.89**

(21) Application number: **84307048.3**

(22) Date of filing: **15.10.84**

(51) Int. Cl.⁴: **B 41 F 15/02,** B 65 D 81/32, B 41 N 1/14

(54) Stencilling device.

(43) Date of publication of application:
23.04.86 Bulletin 86/17

(45) Publication of the grant of the patent:
20.12.89 Bulletin 89/51

(84) Designated Contracting States:
AT BE CH DE FR GB IT LI LU NL SE

(56) References cited:
DE-A-2 107 795
DE-A-2 834 801
FR-A-2 069 786
GB-A-1 081 624
US-A-2 086 011
US-A-3 082 867
US-A-3 221 917
US-A-4 128 057
US-A-4 336 754
US-A-4 374 869
US-A-4 402 402

(73) Proprietor: **Smith, Dwight**
**1641 West 242 Place**
**Harbor City, CA. 90710 (US)**

(72) Inventor: **Smith, Dwight**
**1641 West 242 Place**
**Harbor City, CA. 90710 (US)**

(74) Representative: **Howick, Nicholas Keith et al**
**CARPMAELS & RANSFORD 43 Bloomsbury Square**
**London WC1A 2RA (GB)**

Courier Press, Leamington Spa, England.

## Description

This invention relates to an apparatus for stencilling markings on objects, and more particularly, to such a device employing a compartmentalized pouch having a compartment containing an etching or marking medium (US—A—4,128,057) or such a device containing a different fluid in each of two or more compartments which are joined together to form the marking medium.

The marking of objects with identification markings which may be in the form of numbers, letters or symbols, is used extensively for security purposes. In achieving the desired end result it is highly desirable to employ a marking medium in the form of an etchant or the like which etches the identification symbols into a surface of the object in a manner such that it cannot be readily erased or removed. Effective etchants for metal and glass and removal-resistant paint generally contain substances which can cause injury to the user's skin or eyes. Such materials must therefore be very carefully handled and limited for use by personnel having adequate protection, as well as some skill in their handling. This makes the marking process somewhat more expensive than to be desired, and obviates its use except to specially trained personnel. US—A—2,517,430, issued August 1, 1950, to Hensel et al., describes a typical prior art chemical etching technique.

The method and apparatus of the present invention obviates the aforementioned shortcomings of the prior art in providing a sealed packet containing the fluids needed for etching or painting symbols on a surface which can be employed by an untrained person and without the use of any special protective clothing or gloves.

The improvement is achieved in the present invention by employing a compartmentalized pouch having a plurality of compartments separated by sealing partitions. One of the compartments may contain a first fluid, while an adjacent compartment may contain a second fluid which, when combined with the first, forms an active etching or painting substance. In another embodiment of the invention, a single active fluid may be contained in one of the compartments. The second compartment may also have a stencil forming the identification letters, symbols or markings to be placed on a surface of the object to be marked, this stencil being formed by holes punched through the compartment wall or slots formed in such wall. The stencil is covered over by a tape so that the substance is not released therefrom until the tape is removed and the stencil placed on the surface to be marked. In one embodiment of the invention, one of the fluids forming the etchant or marking substance is contained in the same compartment having the stencil formed thereon, while in another embodiment three compartments are employed, two of the compartments containing the fluids forming the marking substance, while the third compartment has the stencil formed therein. In this second embodiment, the fluids are mixed together in one of the first two compartments and, after mixed to form the marking substance, the fluid is forced through the breakaway sealing divider into the stencilled compartment.

Other types of separator means for forming a sealing separator between the compartments may be employed. Such separator means may comprise a mechnaical clamp which can be mechanically actuated to open the separation between the compartments; a valve installed in the pouch between the compartments; a frangible separator which can easily be broken with finger pressure; a seal which can be punched by an external piercing member; an internal tear seal; etc.

Fig. 1 is a top plan view of a first embodiment of the invention;

Fig. 2 is a cross-sectional view taken along the plane indicated by 2-2 in Fig. 1;

Fig. 3 is a top plan view of a second embodiment of the invention;

Fig. 4 is an exploded view of the second embodiment;

Fig. 5 is a top plan view of a third embodiment of the invention;

Fig. 6 is a cross-sectional view taken along the plane indicated by 6—6 in Fig. 5;

Fig. 7 is a top plan of a first embodiment of the invention;

Fig. 8 is a cross-sectional view taken along the plane indicated by 8—8 in Fig. 7;

Fig. 9 is a top plan view of a second embodiment of the invention;

Fig. 10 is an exploded view of the second embodiment;

Fig. 11 is a top plan view of a third embodiment of the invention;

Fig. 12 is a cross section view taken along the plane indicated by 12—12 in Fig. 11;

Fig. 13 is a perspective view of a clamp which may be employed in implementing the invention;

Fig. 14 is a perspective view of still another clamp device which may be employed;

Fig. 15 is a perspective view showing a further type of clamp device which may be employed;

Fig. 16 illustrates another clamping member which may be employed in the device of the invention;

Fig. 17 illustrates a separator device which may be employed in the device of the invention; and

Fig. 18 illustrates still another type of separator which may be employed.

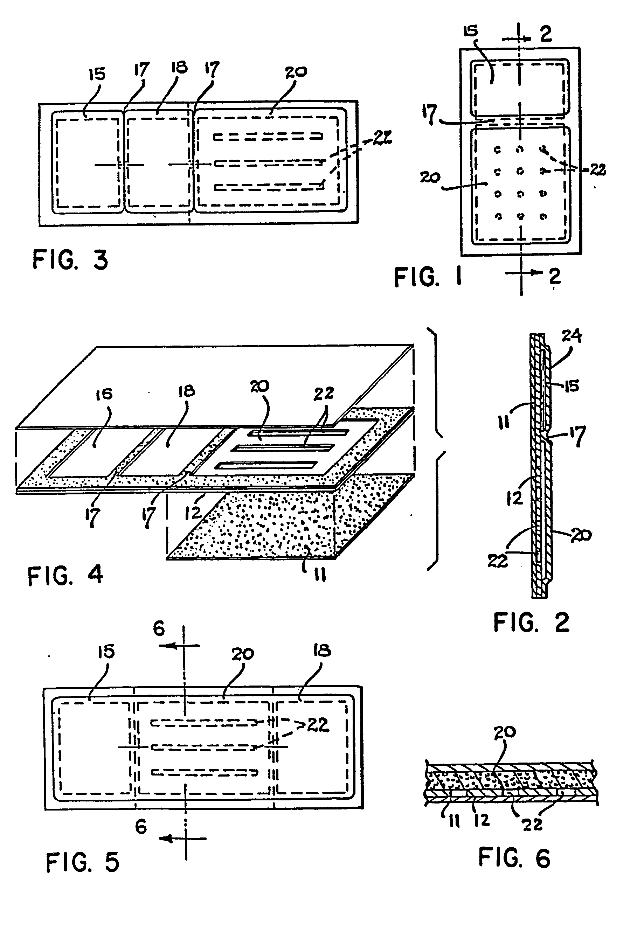

Referring now to Figs 1 and 2, a first embodiment of the invention is illustrated. A pouch member has a first compartment 15 and a second compartment 20 separated by a breakaway seal or septum 17. Typical materials which may be employed for the pouch are 50-gauge polyester film, 0.038mm (.0015) aluminium foil or 0,057mm (.002) polypropylene film. The breakaway seal 17 between the compartments may be fabricated by heat sealing the pouch material sufficiently to form a wall between the compartments which will separate the fluids but which will give way when the compartment walls are compressed. Depending upon the material used, various amounts of heating will be required as is well known in the art. A stencil 22 is formed in the lower wall 12 of compartment 20, this stencil being formed by punched holes or slots incised in wall 12. A sealing tape 11 is removably adhered to wall 12 to cover up the stencil, this layer being removed when the etchant or other marking medium has been squeezed into compartment 20 and is ready for use in marking the object. Wall 12 is preferably coated with a suitable adhesive. The marking substance may be premixed and placed in the compartment 15, or if desired to keep the substance inert until it is ready for use as an etchant, one of the two substances to be combined to form the marking medium is placed in compartment 15 and the other is placed in compartment 20, the two substances being mixed together by squeezing the top wall 24 of compartment 15 to force the fluid through breakaway sealing partition or septum, breaking the septum with the pressure applied thereto and permitting the fluid thus to enter compartment 20 and mix with the other fluid to form the active marking substance.

Various examples of implementations of the invention to form a marking medium are as follows:

### Example I

A mixture of commercial hydrofluoric acid containing 55% by weight of polyvinyl alcohol of the molecular weights and residual acetate composition commercially designated as Gelvatol 20.60, manufactured by Monsanto Corporation was employed. The material was stored in compartment 15 and forced into compartment 20 when stencilling was desired, this material being employed to stencil onto a sheet of safety glass. The stencil was left on the glass for 15 minutes and then removed, the pattern of the stencil being etched into the glass to a depth of 0.076mm (.003 inches)

### Example II

A mixture of ferric chloride and water, available commercially from Phillip Hunt Chemical Company under the label PF Etchant was admixed with the polyvinyl alcohol of Example I and placed in compartment 15. The material in compartment 15 was squeezed through breakaway sealing septum 17 into compartment 20. The tape 11 was removed and the stencil placed on a surface of "1019" steel. After 15 minutes, at room temperature — 20°C, the stencil was removed and the image of the stencil was found to have been etched into the steel to a depth of 0.102mm (.004 inches).

### Example III

A two-component paint, available commercially as NAZDAR gloss black, available from Nazdar Company, Chicago, Illinois was employed, the "A" component of the paint being placed into compartment 15, while the "B" component of the paint was placed into compartment 20. Pressure was exerted on top wall 24 of compartment 15, rupturing breakaway partition 17 and permitting the material in compartment 15 to enter compartment 20. The two components were thoroughly mixed by pushing down on the top wall 24 of compartment 20 in a back and forth rocking action. Tape 11 was removed and the material placed against an aluminium surface to be etched. The stencil was immediately removed and the paint allowed to set.

Referring now to Fig. 4, a second embodiment of the invention is illustrated. In this embodiment, three compartments are employed, the compartments, as in the previous embodiment, being separated from each other by a breakaway sealing septum. First compartment 15 has one of the two fluids contained therein while compartment 18 has the other of the fluids to be mixed with the first fluid to form the marking substance. Compartment 20 has the stencil 22 on its bottom surface, this stencil being sealed by a tape 11 as in the first embodiment. Compartments 15, 18 and 20 are separated from each other by breakaway sealing partition 17, as in the first embodiment. The fluid in compartment 15 is squeezed out of this compartment into compartment 18 where the two fluids are mixed to form the marking substance. When the materials have been thoroughly mixed, the mixture in compartment 18 is squeezed through breakaway partition 17 into compartment 20. The material in compartment 20 is then used to etch the desired surface, as in the previous embodiment.

Referring now to Fig. 5, a further embodiment of the invention is illustrated. This embodiment is similar to that of Fig. 4 except for the fact that compartments 15 and 18, rather than being adjacent to each other, are on opposite ends of compartment 20. In this embodiment, the fluids in compartments 15 and 18 are squeezed into compartment 20 where the mixing thereof is achieved. Otherwise, this embodiment is the same as that of Fig. 4.

The device and technique of the present invention thus can be effectively used for a number of applications by relatively inexperienced personnel. Such applications include the etching of windows and metal parts; the permanent painting of objects made of a variety of different materials, such as plastic, metal glass; the use of the fluid as a transdermal medication; the use of the fluid as a spot test reagent for the identification of metal alloys; the use of the fluid as an antibiotic for testing the susceptibility of an organism etc.

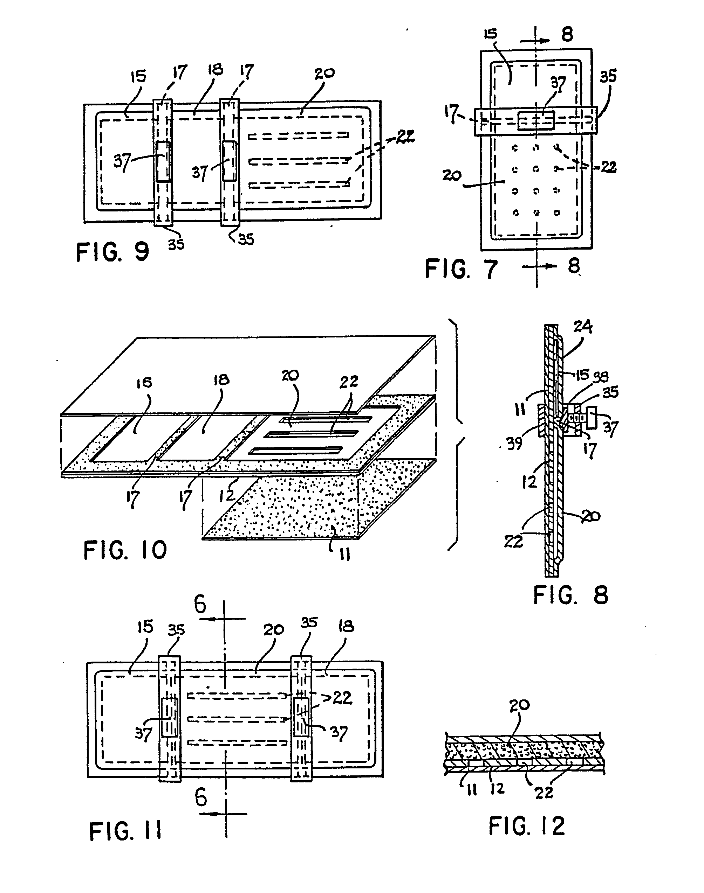

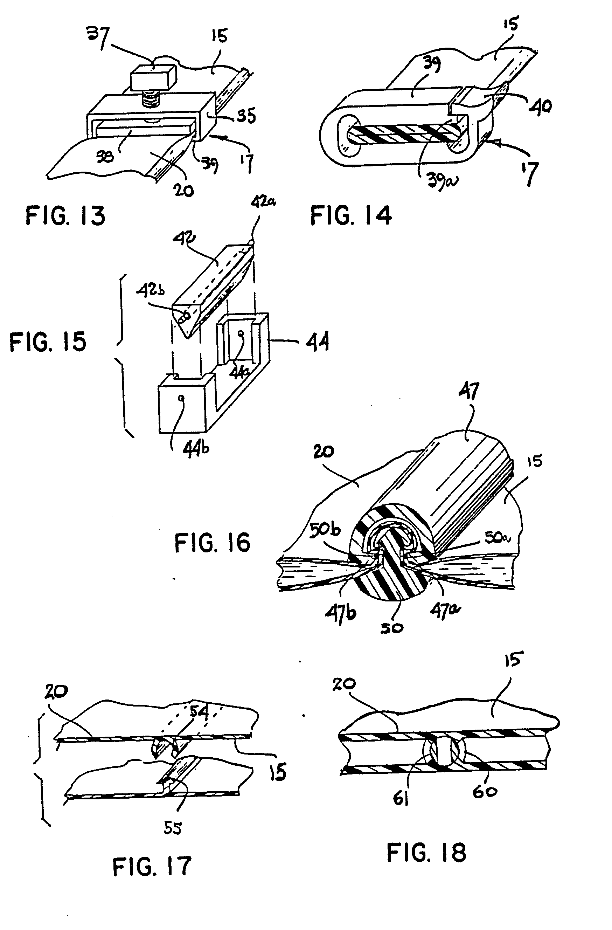

Referring now to Figs 7 and 8, a further embodiment of the invention is illustrated. A pouch member has a first compartment 15 and a second compartment 20 separated from each other by the action of clamp 17 which forms separator means therebetween which can be mechanically released to permit fluid flow between the two compartments. The clamp shown for illustrative purposes is a conventional laboratory type tubing clamp as illustrated in greater detail in Fig. 13 and includes a frame 35, having a screw 37 threadably mounted thereon with a bar member 38 fitted between the screw and the base 39 of the clamp, which clamps the pouch and prevents fluid flow between compartment 15 and 20 when the screw is tightened thereagainst; flow between the compartments being permitted when the screw is loosened. The pouch may be of typical materials specified in connection with the previously described embodiments, with a stencil 22 formed in the lower wall 12 of compartment 20, this stencil being formed by punched holes or slots in inside wall 12. A sealing tape 11 is removably adhered to wall 12 to cover up the stencil, this layer being removed when the etchant or other marking medium is ready for use in marking the object as already described.

Referring now to Fig. 10, a further embodiment of the invention is illustrated, this embodiment being the same as that of Fig. 4 except for the separator means employed. In this embodiment, three compartments are employed, the compartments, as in the previous embodiment, being separated from each other by separator means such as a clamp or the like. The first compartment 15 has one of the two fluids contained therein, while compartment 18 has the other of the fluids to be mixed with the first fluid to form the marking substance. Compartment 20 has stencil 22 on its bottom surface, this stencil being sealed by tape 11 as in the first embodiment. The compartments 15, 18 and 20 are separated from each other by separator means 17 which may comprise a clamp as in the first embodiment. The fluid in compartment 15 may be forced out of this compartment into compartment 18 where the fluids are mixed to form the marking substance. When the materials have been thoroughly mixed, the mixture in compartment 18 is caused to flow into compartment 20. The material in compartment 20 is then used to etch the desired surface, as in the previous embodiment.

Referring now to Fig. 11 and 12, a further embodiment of the invention is illustrated. This embodiment is similar to that of Fig. 10 except for the fact that compartments 15 and 18, rather than being adjacent to each other, are on opposite ends of compartment 20 in this embodiment. The fluids in compartment 15 and 18 are flowed into compartment 20 where the mixing thereof is achieved. Otherwise, this embodiment is the same as that of Fig. 10.

Referring now to Figs. 13—18, various different types of separator means for providing separation between the pouch compartments which can be mechanically actuated to remove such sealing separation are illustrated.

In Fig. 13, a conventional laboratory tubing clamp is shown, this clamp having been already described.

In Fig. 14, a plastic retainer clip mechanism 17 is shown. This mechanism has a resilient arm 39 which has catch 39a which snaps under thumbpiece 40. Thus this clamp mechanism is operated by snapping resilient arm 39 under thumbpiece 40 as shown in the drawing with the clamp being released by downward thumb action on the thumbpiece.

In Fig. 15, an exploded view of a wedge type clamp which comprises a wedge member 42 having pins 42a and 42b on the opposite ends thereof is shown. The pouch member (not shown) is clamped between wedge member 42 and holder member 44 with the wedge being retained in the holder by means of pins 42a and 42b which fit into mating apertures 44a and 44b formed in holder member 44.

Fig. 16 illustrates still another type of clamping member which may be employed for forming a separator means between compartments 15 and 20. This clamping member has a semi-circular slide clamping member 47 which has a pair of rails 47a and 47b which ride in tracks 50a and 50b respectively formed in mating clamp member 50. The pouch is effectively clamped between members 47 and 50 at the opposing faces of rails 47a and 47b and tracks 50a, 50b respectively.

Referring now to Fig. 17, separator means in the form of a ziploc fastener is illustrated, the separator means being formed by interlocking members 54 and 55 of the fastener, compartments 15 and 20 being formed on opposite sides of this closure.

Referring now to Fig. 18, still another type of separator means which may be employed is illustrated, this separator means being in the form of a valve having a valve body 60 embedded in the pouch between compartments 15 and 20 and having a rotatable valve cock 61 installed therein which can be manually rotated to either prevent fluid communication between compartments 15 and 20 or to permit such fluid communication.

It should be apparent that many other additional types of separator means for achieving the desired end results in applicant's device can be employed such as, for example, a frangible glass or plastic member which collapses and permits fluid flow when manually pinched, as described in US Patent No: 4,183,684; a rupturable diaphragm as described in US Patent No: 3,301,390, a device that is internally punctured by the application of manual pressure such as described in US Patent No: 2,907,173; an internal tear seal device such as described in US Patent No: 3,294,227; etc.

Thus, it should be apparent many different types of separator means can be employed which provides sealing separation between the two compartments and which can be mechanically actuated to remove the sealing separation to implement the operation of the device of the invention.

While the device of the invention has been described and illustrated in detail, it is to be clearly understood that this is intended by the way of illustration and example only and is not to be taken by way of limitations, the scope of this invention being limited only by the terms of the following claims.

## Claims

1. A pouch for use in placing a fluid onto the surface of an object, the pouch comprising walls (12, 24) defining a compartment (15) containing a fluid, one of said walls (12) having at least one stencil opening therein, characterised in that said walls (12, 24) define also a second compartment (20) having the portion of said wall (12) with said stencil opening therein, removable means (11) are attached to said wall (12) with said stencil opening; and separator means (17) are provided between said compartments (15, 20) to form a seal therebetween, said separator means (17) being capable of mechanical actuation to remove the sealing separation whereby the fluid in the first-mentioned compartment (15) can be caused to enter said second compartment (20) and to pass through said stencil opening onto the surface of an object against which said wall (12) with the stencil opening is placed.

2. A pouch according to claim 1 wherein said stencil opening is in the form of a predetermined image to be marked on the surface of the object, said fluid being a marking fluid.

3. A pouch according to claim 2 wherein said second compartment (20) also contains a fluid, the fluids being of a kind which are admixed with each other to form the marking fluid.

4. A pouch according to any one of the preceding claims wherein said separator means (17) comprises mechanical clamping means (Figs. 13—17).

5. A pouch according to any of claims 1—3 wherein said separator means comprises valve means (Fig. 18).

6. A pouch according to any of claims 1—3 wherein the separator means (17) comprises a breakaway partition which breaks when the walls defining said first compartment are squeezed.

7. A pouch according to claim 2 wherein a third compartment (18) is formed in said pouch, said third compartment containing a fluid to be admixed with the fluid in said first compartment (15) to form the marking fluid.

8. A pouch according to claim 7 wherein the third compartment (18) is adjacent to said first compartment (15).

9. A pouch according to claim 7 wherein said third compartment (18) is adjacent to said second compartment (20) and on a side thereof opposite to said first compartment (15).

10. A pouch according to claim 2 wherein the marking fluid is a chemical etchant.

11. A pouch according to claim 3 wherein the marking fluid is a two-component paint.

12. A pouch according to claim 1 wherein said fluid is a transdermal medication.

13. A pouch according to claim 1 wherein said fluid is a spot test reagent for identifying metal alloys.

14. A pouch according to claim 1 wherein the fluid is an antibiotic.

## Patentansprüche

1. Beutel zum Anordnen eines Fluids auf der Oberfläche eines Objektes, wobei der Beutel Wände (12, 24) aufweist, die ein Abteil (15) begrenzen, das ein Fluid enthält, wobei eine der Wände (12) wenigstens eine Schablonenöffnung aufweist, dadurch gekennzeichnet, daß die Wände (12, 24) auch ein zweites Abteil (20) begrenzen, das den Abschnitt der Wand (12) mit der Schablonenöffnung darin aufweist, daß eine entfernbare Einrichtung (11) an der Wand (12) mit der Schablonenöffnung befestigt ist, daß eine Trenneinrichtung (17) zwischen den Abteilen (15, 20) vorgesehen ist, um eine Dichtung dazwischen zu bilden, und daß durch mechanische Betätigung der Trenneinrichtung (17) die dichtende Trennung entfernbar ist, wodurch das Fluid aus dem ersten Abteil (15) in das zweite Abteil (20) eintreten und durch die Schablonenöffnung auf die Oberfläche eines Objektes gelangen kann, gegen welches die Wand (12) mit der Schablonenöffnung angeordnet ist.

2. Beutel nach Anspruch 1, wobei die Schablonenöffnung in Form eines vorbestimmten Bildes ausgebildet ist, das auf der Oberfläche des Objektes auszubilden ist, und das Fluid ein Markierungsfluid ist.

3. Beutel nach Anspruch 2, wobei das zweite Abteil (20) ebenfalls ein Fluid enthält und die Fluide von einer Art sind, welche zusammengemischt werden, um das Markierungsfluid zu bilden.

4. Beutel nach einem der vorhergehenden Ansprüche, wobei die Trenneinrichtung (17) eine mechanische Klemmeinrichtung aufweist (Fig. 13 bis 17).

5. Beutel nach einem der Ansprüche 1 bis 3, wobei die Trenneinrichtung eine Ventileinrichtung aufweist (Fig. 18).

6. Beutel nach einem der Ansprüche 1 bis 3, wobei die Trenneinrichtung (17) eine Aufreißtrennwand aufweist, welche zerreißt, wenn die Wände, die das erste Abteil begrenzen, gequetscht werden.

7. Beutel nach Anspruch 2, wobei ein drittes Abteil (18) in dem Beutel ausgebildet ist, und wobei das dritte Abteil ein Fluid enthält, das dem Fluid in dem ersten Abteil (15) zugemischt wird, um das Markierungsfluid zu bilden.

8. Beutel nach Anspruch 7, wobei das dritte Abteil (18) an das erste Abteil (15) angrenzt.

9. Beutel nach Anspruch 7, wobei das dritte Abteil (18) an das zweite Abteil (20) an einer Seite von diesem gegenüber dem ersten Abteil (15) angrenzt.

10. Beutel nach Anspruch 2, wobei das Markierungsfluid ein chemisches Ätzmittel ist.

11. Beutel nach Anspruch 3, wobei das Markierungsfluid eine Zweikomponentenfarbe ist.

12. Beutel nach Anspruch 1, wobei das Fluid ein durch die Haut dringendes Arzneimittel ist.

13. Beutel nach Anspruch 1, wobei das Fluid ein Tropfenprobenreagenz zur Bestimmungen von Metallegierungen ist.

14. Beutel nach Anspruch 1, wobei das Fluid ein Antibiotikum ist.

**Revendications**

1. Sachet destiné à être utilisé pour appliquer un fluide sur la surface d'un objet, le sachet comportant des parois (12, 24) délimitant un compartiment (15) renfermant un fluide, une desdites parois (12) ayant au moins une ouverture de pochoir, caractérisé en ce que lesdites parois (12, 24) délimitent également un second compartiment (20) comportant la zone de ladite paroi (12) présentant ladite ouverture de pochoir, des moyens amovibles (11) sont fixés à ladite paroi (12) avec ladite ouverture de pochoir; et des moyens séparateurs (17) sont prévus entre lesdits compartiments (15, 20) pour former une structure étanche entre eux, lesdits moyens séparateurs (17) étant suceptibles d'être actionnés mécaniquement pour supprimer la séparation étanche, de sorte que le fluide dans le premier compartiment spécifié (15) peut être amené à pénétrer dans ledit second compartiment (20) et à passer par ladite ouverture de pochoir sur la surfaoe d'un objet contre lequel est disposée ladite paroi (12) avec l'ouverture de pochoir.

2. Sachet selon revendication 1, dans lequel ladite ouverture de pochoir a la forme d'une image prédéterminée destinée à être inscrite sur la surface d'un object, ledit fluide étant un fluide de marquage.

3. Sachet selon la revendication 2, dans lequel ledit second compartiment (20) renferme aussi un fluide, les fluides étant d'une nature telle qu'ils peuvent se mélanger l'un avec l'autre pour forme un fluide de marquage.

4. Sachet selon l'une des revendications précédentes dans lequel lesdits moyens séparateurs (17) comportent des moyens mécaniques des serrage (figure 13, 17).

5. Sachet selon l'une des revendications 1 à 3, dans lequel lesdits moyens séparateurs comportent des moyens formant vanne (figure 18).

6. Sachet selon l'une quelconque des revendications 1 à 3, dans lequel les moyens de séparation (17) comportent une cloison de fracture qui se rompt lorsque les parois délimitant ledi't premier compartiment sont pressées.

7. Sachet selon la revendication 2, dans lequel un troisième compartiment (18) est ménagé dans ledit sachet, ce troisième compartiment renfermant un fluide destiné à être mélangé avec le fluide dans ledit premier compartiment (15) pour former le fluide de marquage.

8. Sachet selon la revendication 7, dans lequel le troisième compartiment (18) est adjacent audit premier compartiment (15).

9. Sachet selon la revendication 7, dans lequel ledit troisième compartiment (18) est adjacent audit second compartiment (20) et à un des côtés de ce dernier en regard dudit premier compartiment (15).

10. Sachet selon la revendication 2, dans lequel le fluide de marquage est un agent de gravure chimique.

11. Sachet selon la revendication 3, dans lequel le fluide de marquage est une peinture à deux constitutants.

12. Sachet selon la revendication 1, dans lequel ledit fluide est un médicament intradermique.

13. Sachet selon la revendication 1, dans lequel ledit fluide est un réactif d'essai à la goutte pour identifier des alliages métalliques.

14. Sachet selon la revendication 1, dans lequel le fluide est un antibiotique.

FIG. 3

FIG. 1

FIG. 4

FIG. 2

FIG. 5

FIG. 6

FIG. 9

FIG. 7

FIG. 10

FIG. 8

FIG. 11

FIG. 12

FIG. 13

FIG. 14

FIG. 15

FIG. 16

FIG. 17

FIG. 18

3